# EUROPÄISCHE PATENTSCHRIFT

(11) **EP 1 757 265 B1**
(45) Veröffentlichungstag und Bekanntmachung des Hinweises auf die Patenterteilung: **29.04.2009**
(21) Anmeldenummer: 06017028.9
(22) Anmeldetag: 16.08.2006
(51) Int. Cl.: A61K 8/67, A61K 8/97, A61Q 19/00

(54) **Kosmetikum mit einem Ascorbinsäure-haltigem Inhaltsstoff aus Sorbus torminalis**
Cosmetic composition comprising ascorbic acid or derivative from sorbus torminalis
Composition cosmétique comprenant vitamin c ou ses derivés de sorbus torminalis

(30) Priorität: 22.08.2005 DE 102005039819
(43) Veröffentlichungstag der Anmeldung: 28.02.2007
(73) Patentinhaber: Dr. Babor GmbH & Co. KG, 52078 Aachen (DE)
(72) Erfinder: Neumann, Elmar, 52152 Simmerath (DE); Rietfort, Hans-Dieter, 52078 Aachen (DE); Weber, Andrea, 42653 Solingen (DE); Von Brocke, Alexander, Dr., 52076 Aachen (DE)
(74) Vertreter: Bauer, Dirk

(56) Entgegenhaltungen:
- WO-A-01/17495
- WO-A2-03/086329
- TSITSA-TZARDI E; LOUKIS A: "Constituents of Sorbus torminalis fruits" FITOTERAPIA, Bd. 62, Nr. 3, 1991, Seiten 282-283, XP009075172

## Beschreibung

Die Erfindung betrifft ein Kosmetikum mit Ascorbinsäure-haltigem Inhaltsstoff. Kosmetika sind Mittel zur äußerlichen Anwendung zum Zwecke der Reinigung, der Pflege und des Schutzes der (menschlichen) Haut. Insbesondere fallen darunter Seifen, Syndets und flüssige tensidhaltige Produkte wie Wasch-, Dusch- und Haarwaschmittel sowie Badezusätze, kosmetische Hautpflegemittel auf Basis von Emulsionen, Gelen, Schäumen, Ölen und Wässern, Lichtschutzpräparate und Duftmittel wie EdT, Parfums, Deodorantien und Antitranspirantien.

Ascorbinsäure (auch: "Vitamin C") und deren Salze finden in Kosmetika in allgemein bekannter Weise Verwendung als so genannte Radikalfänger: Sie vermindern die schädigenden Wirkungen freier Radikale, die einerseits unter Stress und andererseits unter langwelliger UV/A-Bestrahlung als Teil des natürlichen Sonnenlichts oder in Solarien in tieferen Schichten der Haut gebildet werden und wirken so einem der Hauptfaktoren der Hautalterung entgegen, insbesondere den (bisweilen unter dem Begriff des "Foto-Aging" zusammengefassten) Symptomen der Austrocknung und Verlederung der Haut, der Erweiterung von oberflächennahen Blutgefäßen und der Bildung von Pigmentflecken.

### Aufgabe

Der Erfindung liegt die Aufgabe zugrunde, ein alternatives Kosmetikum vorzuschlagen.

### Lösung

Ausgehend von den bekannten Kosmetika wird nach der Erfindung vorgeschlagen, dass der Inhaltsstoff ein Elsbeerextrakt ist.

Der Els- oder Elzbeerbaum "Sorbus torminalis L. Crantz", auch bekannt als Aris- oder Arlesbeere, Ruhrbirne, Atlasbaum, Schweizer Birn- oder Wilder Sperberbaum gehört wie die bekanntere Vogelbeere (Sorbus aucuparia) nach der von Carl von Linné vorgeschlagenen Systematik zur Gattung Sorbi (Mehlbeeren) in der Familie der Rosaceae (Rosengewächse), Unterfamilie Maloideae (Kernobstgewächse). In der Literatur wird der Begriff "Elsbeere" sowohl für den Baum wie auch für seine Frucht verwendet. Im Rahmen dieser Schrift wird zur begrifflichen Unterscheidung der Baum als "Elsbeerbaum" bezeichnet.

Die medizinische Wirksamkeit der Inhaltsstoffe der Elsbeere ist allgemein bekannt: Sie findet - wie die Vogelbeere - in der Naturheilkunde Verwendung beispielsweise zur Behandlung von Durchfallerkrankungen (daher der Name "Ruhrbirne"). Die Elsbeere weist neben einem hohen Gehalt an Ascorbinsäure signifikante Mengen von (in Pflanzen fast ubiquitären) Flavonoiden auf, die allgemein für die pharmazeutisch relevante Wirkung zahlreicher pflanzlicher Extrakte von einer diuretischen, venenverstärkenden Wirkung bis hin zur Unterstützung enzymatischer Prozesse und der Wirkung von Ascorbinsäure als relevant gelten. Darüber hinaus enthält die Elsbeere Zuckeralkohole, die in Kosmetika eine Zunahme der Feuchtigkeit in der Haut begünstigen. Abgesehen von den kosmetisch wirksamen Inhaltsstoffen der Frucht, die eine besonders gute Eignung der Elsbeere zur Herstellung eines Inhaltsstoffs eines Kosmetikums begründen, weist der markante sommergrüne, 15 bis 25 m hohe Elsbeerbaum durch sein regional begrenztes Vorkommen insbesondere in der Eifel ein auch mit Blick auf Natürlichkeit und Naturverbundenheit werbetechnisch nutzbares hohes lokales Identifikationspotential auf.

Die Einsatzkonzentration des Inhaltsstoffs in einem erfindungsgemäße Kosmetikum beträgt vorzugsweise zwischen 0,5 und 7 %, insbesondere zwischen 1 und 5 %.

Besonders bevorzugt ist der Inhaltsstoff eines erfindungsgemäßen Kosmetikums ein Mazerat oder ein Wasserdampf-Destillat, also ein Produkt aus einem Mazerations- oder aus einem Wasserdampf-Destillationsverfabren. Gegenüber den übrigen Extraktionsverfahren des Arzneibuchs, der Prekolation, der Fluidextraktion und der zähflüssigen oder Dickextraktion sowie gegenüber anderen allgemein bekannten Extraktionsverfahren (beispielsweise durch überkritische Phasen oder mittels CO₂) sind diese beiden besonders geeignet, die kosmetisch wirksamen Inhaltsstoffe der Elsbeere schonend und zugleich wirtschaftlich zu extrahieren.

### Ausführungsbeispiel

Die Erfindung wird nachfolgend anhand von Ausführungsbeispielen erläutert.

Zur Herstellung eines Mazerats als Inhaltsstoff für eine Serie von erfindungsgemäßen Kosmetika zur Hautreinigung und -pflege werden reife Elsbeeren gewaschen und die Stängel entfernt. Die ganzen Beeren werden mit gereinigtem Wasser als Extraktionsmittel in einem so genannten "kalten Aufguss" in ein geeignetes Gefäß gegeben. Das Gefäß wird verschlossen für einige Zeit stehen gelassen und in regelmäßigen Abständen durchmischt. Wenn die kosmetisch wirksamen Bestandteile in die Extraktionsflüssigkeit in Lösung gegangen sind, wird diese von dem Rückstand getrennt.

In einer anderen Ausführung werden zur Herstellung eines Wasserdampf-Destillats als Inhaltsstoff für eine weitere Serie von erfindungsgemäßen Kosmetika reife Elsbeeren gewaschen, entkernt, von Stängeln befreit, zerkleinert und mit Wasserdampf versetzt. Hierbei gehen die kosmetisch wirksamen Bestandteile in den Wasserdampf über, der anschließend in einem weiteren Gefäß aufgefangen wird.

Der jeweils gewonnene Extrakt wird gegebenen Falls filtriert und konserviert. Die Analytik der Leitsubstanzen erfolgt über HPLC und DC, die Einsatzkonzentration des jeweiligen Extrakts wird über den Ascorbinsäuregehalt eingestellt.

## Patentansprüche

1. Kosmetikum mit Ascorbinsäure-haltigem Inhaltsstoff, ***dadurch gekennzeichnet, dass*** der Inhaltsstoff ein Elsbeerextrakt ist, wobei eine Einsatzkonzentration des Inhaltsstoffs in dem Kosmetikum zwischen 0,5 und 7 % beträgt.

2. Kosmetikum nach dem vorgenannten Anspruch, ***dadurch gekennzeichnet, dass*** die Einsatzkonzentration zwischen 1 und 5 % beträgt.

3. Kosmetikum nach einem der vorgenannten Ansprüche, ***dadurch gekennzeichnet, dass*** der Inhaltsstoff ein Mazerat ist.

4. Kosmetikum nach einem der Ansprüche 1 oder 2, ***dadurch gekennzeichnet, dass*** der Inhaltsstoff ein Wasserdampf-Destillat ist.

## Claims

1. A cosmetic with a substance containing ascorbic acid, **characterised in that** the substance is wild service tree extract, wherein the application concentration of the substance in the cosmetic is between 0.5 and 7 %.

2. The cosmetic according to the aforementioned claim, **characterised in that** the application concentration is between 1 and 5 %.

3. The cosmetic according to one of the preceding claims, **characterised in that** the substance is a macerate.

4. The cosmetic according to one of the claims 1 or 2, **characterised in that** the substance is a steam distillate.

## Revendications

1. Produit cosmétique dont un composant contient de l'acide ascorbique, **caractérisé en ce que** ledit composant est un extrait de baies de l'alisier torminal, ledit composant pouvant être mis en oeuvre de façon à ce que sa concentration dans le produit cosmétique soit comprise entre 0,5 et 7 %.

2. Produit cosmétique selon la revendication précédente, **caractérisé en ce que** ladite concentration de mise en oeuvre est comprise entre 1 et 5 %.

3. Produit cosmétique selon l'une des revendications précédentes, **caractérisé en ce que** ledit composant a été obtenu par macération.

4. Produit cosmétique selon l'une des revendications 1 ou 2, **caractérisé en ce que** ledit composant a été obtenu par hydrodistillation.
